(19) Europäisches Patentamt
European Patent Office
Office européen des brevets

(11) Veröffentlichungsnummer: **0 028 337 B1**

(12) # EUROPÄISCHE PATENTSCHRIFT

(45) Veröffentlichungstag der Patentschrift: **13.02.85**

(51) Int. Cl.⁴: **C 07 C 118/00,** C 07 C 119/048, C 07 C 125/073, C 07 C 125/075

(21) Anmeldenummer: **80106297.7**

(22) Anmeldetag: **16.10.80**

(54) **Verfahren zur Herstellung von Mischungen aus Diphenylmethan-diisocyanaten und Polyphenyl-polymethylen-polyisocyanaten.**

(30) Priorität: **20.10.79 DE 2942542**

(43) Veröffentlichungstag der Anmeldung: **13.05.81 Patentblatt 81/19**

(45) Bekanntmachung des Hinweises auf die Patenterteilung: **13.02.85 Patentblatt 85/7**

(84) Benannte Vertragsstaaten: **BE DE FR GB IT NL**

(56) Entgegenhaltungen:
**EP - A - 0 018 581**
**EP - A - 0 018 583**
**US - A - 2 806 051**

(73) Patentinhaber: **BASF Aktiengesellschaft, Carl-Bosch-Strasse 38, D-6700 Ludwigshafen (DE)**

(72) Erfinder: **Merger, Franz, Dr., Max-Slevogt-Strasse 25, D-6710 Frankenthal (DE)**
Erfinder: **Nestler, Gerhard, Dr., Rubenstrasse 25, D-6700 Ludwigshafen (DE)**
Erfinder: **Towae, Friedrich, Dr., Parkstrasse 22, D-6700 Ludwigshafen (DE)**
Erfinder: **Harder, Wolfgang, Dr., Loensstrasse 20, D-6940 Weinheim (DE)**

**EP 0 028 337 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Mischungen aus Diphenylmethan-diisocyanaten und Polyphenyl-polymethylen-polyisocyanaten (roh-MDI) durch Umsetzung von

A) Anilin mit O-Alkylcarbamidsäureestern und gegebenenfalls Harnstoff und Alkoholen, vorzugsweise in Gegenwart von Katalysatoren, zu N-Phenylurethanen,
B) Kondensation der erhaltenen N-Phenylurethane mit Formaldehyd, formaldehydabspaltenden Verbindungen oder bifunktionellen Verbindungen der Formel $X-CH_2-X$, wobei X einen RO-, RS- oder ROCO-Rest und R einen Alkylrest bedeuten, in Gegenwart von Säuren oder Carbonsäuren zu Mischungen aus Methylen-bis-phenylurethanen und Polymethylen-polyphenylurethanen (roh-MDU) und
C) thermische Spaltung des roh-MDU bei 175 bis 600° C, vorzugsweise in flüssiger Phase bei Temperaturen von 175 bis 350° C und vorzugsweise in Gegenwart eines in heterogener Phase vorliegenden Metalls aus der Reihe Zink, Aluminium, Titan, Eisen, Chrom, Kobalt und Nickel als Katalysator.

Zur Herstellung von Mischungen aus Diphenylmethan-diisocyanaten und Polyphenyl-polymethylen-polyisocyanaten — im folgenden kurz roh-MDI genannt — werden üblicherweise Mischungen aus Diphenylmethan-diaminen und Polyphenyl-polymethylen-polyaminen — im folgenden kurz roh-MDA genannt — mit Phosgen in die entsprechenden Carbamidsäurechloride übergeführt und diese danach thermisch, gegebenenfalls in Gegenwart von Katalysatoren, gespalten.

Roh-MDA wird seinerseits durch Kondensation von Anilin mit Formaldehyd in Gegenwart von sauren Katalysatoren z. B. gemäß BE-PS 648 787 und CA-PS 700 026 hergestellt. In der Technik setzt man große Mengen Salzsäure als Katalysator ein, die üblicherweise nach der Kondensation mit Basen neutralisiert und aus dem Reaktionsgemisch abgetrennt wird. Problematisch sind besonders: der hohe Umsatz von Chlor über Phosgen und Carbamidchloride in Chlorwasserstoff, die Toxizität des Phosgen und die Korrosivität der Reaktionsgemische, verbunden mit kostspieligem Material- und sicherheitstechnischem Aufwand.

Die chlorfreie, edelmetallkatalysierte, technisch ebenfalls problematische Direktsynthese Isocyanaten aus Nitroaromaten und Kohlenmonoxid z. B. nach GB-PS 1 025 436, DE-OS 1 815 517 (US-PS 3 576 835), DE-OS 1 931 212 (US-PS 3 654 279 und 3 781 321) kommt für roh-MDI nach dem gegenwärtigen Stand der Technik schon deshalb nicht in Betracht, da es eine geeignete Synthese zur Herstellung von Polynitro-polyphenyl-methanen nicht gibt.

Man hat vorgeschlagen, zur Herstellung von Isocyanaten Urethane, die als verkappte Isocyanate anzusehen sind, thermisch zu spalten.

Eine Synthese von Methylen-bis-phenylurethan und Polymethylen-polyphenylurethan (roh-MDU) ist nach DE-PS 1 042 891 (US-PS 2 946 768) durch Kondensation von Phenylurethan mit Formaldehyd möglich. Das beschriebene Verfahren führte jedoch zu keinem technischen Erfolg, da

— zur Herstellung des Phenylurethans die Umsetzung von Anilin mit Chlorkohlensäureester bzw. von Phenylisocyanat mit Alkohol empfohlen wird und damit die Probleme der Verwendung von Phosgen nicht eliminiert waren und
— die Kondensationsprodukte aus Phenylurethan und Formaldehyd nach DE-PS 1 042 891 Hydrolyseprodukte mit freien Aminogruppen und beträchtliche Anteile (15 bis 50%) N—C-verknüpfter Komponenten enthalten, die nicht zu roh-MDI gespalten werden können.

Aus der US-PS 2 409 712 und US-PS 2 806 051 war zwar ein chlorfreies Verfahren zur Herstellung von Phenylurethan durch Reaktion von Anilin, Harnstoff und Alkohol bekannt; es war jedoch wegen der relativ mäßigen Ausbeuten im Vergleich zur Phosgenierung von Anilin und anschließender Umsetzung mit Alkohol nicht erfolgversprechend und wurde daher in später beschriebenen Verfahren zur Urethanherstellung überhaupt nicht mehr erwähnt.

Auch das in der DE-OS 2 160 111 (US-PS 3 763 217) beschriebene Verfahren zur Herstellung von N-substituierten Urethanen, z. B. Phenylurethan durch Umsetzung eines organischen Carbonats mit Anilin in Gegenwart einer Lewis-Säure brachte keinen entscheidenden Fortschritt. Nachteilig an diesem Verfahren ist, daß Dialkylcarbonat aus Phosgen und Alkohol oder durch die technisch noch problematische katalytische Cooxidation Von Kohlenmonoxid und Alkohol hergestellt werden muß und noch teuer ist, die Reaktionsgeschwindigkeit recht gering und die Selektivität infolge konkurrierender Bildung von N-Alkyl-anilinen unbefriedigend sind.

Nach jüngeren Vorschlägen können Arylurethane ohne Phosgen aus Nitroaromaten, Alkohol und Kohlenmonoxid gewonnen und so mit besseren Erfolgschancen in Isocyanate übergeführt werden (GB-PS 1 247 451).

Nach Angaben der DE-OS 1 568 044 (US-PS 3 467 694) werden Urethane durch Umsetzung von organischen Nitroverbindungen, Kohlenmonoxid und hydroxylhaltigen Verbindungen in Gegenwart eines Katalysators, der aus einem Edelmetall und einer Lewissäure besteht, unter praktisch wasserfreien Bedingungen in Abwesenheit von Wasserstoff bei erhöhtem Druck und Temperaturen über

150°C hergestellt. Gemäß DE-OS 2 343 826 (US-PS 3 895 054) werden Urethane aus Hydroxylgruppenhaltigen Verbindungen, Kohlenmonoxid und Nitro-, Nitroso-, Azo- und Azoxygruppen aufweisenden Verbindungen in Gegenwart von Schwefel, Selen, einer Schwefel- und/oder Selenverbindung und mindestens einer Base und/oder Wasser erhalten. Die DE-OS 2 623 694 (US 4 080 365) beschreibt die Herstellung aromatischer Urethane aus den oben genannten Ausgangsverbindungen in Gegenwart von Selen enthaltenden Katalysatorsystemen sowie speziellen aromatischen Amino- und Harnstoffverbindungen.

Aber auch diese Verfahren weisen noch schwerwiegende Nachteile auf. Das toxische Kohlenmonoxid und Katalysatoren, die toxisch sind oder im Laufe der Umsetzung toxische Verbindungen bilden, beispielsweise Selen- und Schwefelwasserstoff oder Katalysatoren, die sehr teuer und schwierig rückführbar sind, wie Palladium, erfordern einen hohen technischen Aufwand und kostspielige Sicherheitsmaßnahmen.

Die Verarbeitung des so herstellbaren Phenylurethans mit Formaldehyd analog der oben genannten DE-PS 1 042 891 liefert nach DE-OS 2 832 379 ein besser geeignetes roh-MDU, wenn das erhaltene Kondensat entwässert und zur Vervollständigung der Umlagerung von N—C- zu C—C-verknüpften Produkten mit Säuren behandelt wird. Die verwendeten großen Säuremengen und gebildeten Nebenprodukte, z. B. Amine, stellen eine bedeutende Abwasserbelastung dar. Die verbesserte Produktqualität des roh-MDU gemäß DE-OS 2 832 379 muß daher durch einen weiteren erhöhten technischen Aufwand erkauft werden.

Wie bereits dargelegt wurde, sind N-substituierte Urethane anschließend thermisch in Isocyanate spaltbar. Bei der thermischen Spaltung treten gleichzeitig verschiedene unerwünschte Nebenreaktionen auf. Genannt seien beispielsweise: die Decarboxylierungsreaktion der Urethane, die von der Bildung primärer und sekundärer Amine sowie Olefine begleitet sein können; Umsetzungen zwischen dem gebildeten Isocyanat und Urethan zu Allophanaten bzw. Amin zu Harnstoffen und Polymerisation der Isocyanate zu Isocyanuraten.

Die Pyrolyse der Urethane in der Dampfphase — Dampfphase ist hierbei so definiert, daß das Produktgemisch, gegebenenfalls einschließlich des Lösungsmittels, nach der Spaltung in der Dampfphase vorliegt, unabhängig davon, ob die zu spaltenden Urethane gasförmig, flüssig oder fest zugeführt werden — wird nach Angaben der DE-OS 1 944 719 (GB-PS 1 247 451) bei Temperaturen von 400 bis 600°C in Gegenwart einer Lewissäure als Katalysator durchgeführt, wobei das Isocyanat und der Alkohol durch fraktionierte Kondensation getrennt wird. Toluylendiisocyanat wird z. B. durch Pyrolyse von Toluylen-2,4-diäthylurethan in Gegenwart von Eisen(III)-chlorid erhalten. Nachteile der Reaktion sind u. a. niedrige Ausbeuten verbunden mit beträchtlichen Mengen eines polymeren Nebenprodukts, Zersetzung und Korrosivität des Katalysators. In der DE-OS 2 410 505 (US 3 870 739) wird ein Verfahren beschrieben, bei dem das Urethan bei einer Temperatur von 350 bis 550°C und einem Druck von weniger als dem (p + 1)fachen des Isocyanatdampfdruckes in einer katalysatorfreien Pyrolysezone innerhalb von 15 Sekunden gespalten wird. Nachteilig an diesem Verfahren ist u. a., daß eine große für die endotherme Spaltung erforderliche Wärmemenge innerhalb einer sehr kurzen Zeit dem pulverförmigen Urethan zugeführt werden muß und ein als Nebenprodukt anfallendes festes Polymer und dessen Abtrennung die Durchführung eines kontinuierlichen Verfahrens erschwert.

Die thermische Spaltung von Urethanen in flüssiger Phase wird beispielsweise in den DE-AS 2 421 503 (US 3 962 302) und DE-AS 2 530 001 (US 3 919 280) beschrieben. Nach Angaben der DE-AS 2 421 503 werden die Urethane in einem inerten Lösungsmittel, wie Alkylbenzolen, linearen und cyclischen Kohlenwasserstoffen und/oder Phthalsäureestern, gelöst und unter Normal- oder Überdruck bei 175 bis 350°C gespalten. Die Isolierung und Trennung des erhaltenen Isocyanats und Alkohols erfolgt mit Hilfe des Lösungsmittels als Trägermittel und/oder unter Verwendung eines Inertgases als Trägermittel. Gemäß DE-AS 2 530 001 werden als Reaktionsmedium höhermolekulare, gegebenenfalls substituierte aliphatische, cycloaliphatische oder aromatische Kohlenwasserstoffe, Äther, Ester oder Ketone verwendet. Zur Trennung der Spaltprodukte wird nur die Destillation genannt, wobei über Kopf Isocyanat, Alkohol und Trägermaterial abdestilliert werden, während das Reaktionsmedium als Bodenfraktion zurückbleibt.

Zur Herstellung von aromatischen Isocyanaten werden die Urethane nach DE-OS 2 635 490 bei Temperaturen von 150 bis 350°C unter vermindertem Druck mit einer Lösung aus wenigstens einem Metallion, wie Ionen von Kupfer, Zink, Aluminium, Zinn, Titan, Vanadium, Eisen, Kobalt und Nickel als Katalysator, der in einem inerten Lösungsmittel mit einem Siedepunkt von 200°C in einer Metallkonzentration von wenigstens 0,001 Gew.-%, bezogen auf das Lösungsmittel, gelöst ist, in Kontakt gebracht. Die Trennung der erhaltenen Spaltprodukte erfolgt durch fraktionierte Kondensation. Nach den genannten Methoden können Urethane in Abhängigkeit von ihrer Struktur in teilweise sehr guten Ausbeuten in Isocyanate übergeführt werden. Nicht beispielhaft beschrieben in diesen Patentschriften wird die Herstellung von roh-MDI.

Im Unterschied zu den beispielhaft genannten Isocyanaten ist roh-MDI nicht mit Hilfe von Lösungsmitteln als Träger vollständig destillierbar und kann daher nicht wie beschrieben vom Katalysator und gegebenenfalls nicht umgesetzten Ausgangsstoffen sowie von Verunreinigungen isoliert werden.

Die Aufgabe der vorliegenden Erfindung bestand darin, roh-MDI wirtschaftlich und ökologisch vorteilhaft aus leicht zugänglichen Ausgangsstoffen ohne die toxischen Komponenten Phosgen, Kohlenmonoxid, Chlor und Chlorwasserstoff und ohne kostspielige bzw. toxische Katalysatoren, wie Palladium oder Selen, zugänglich zu machen.

Die Aufgabe wurde gelöst durch ein Verfahren zur Herstellung von Mischungen aus Diphenylmethan-diisocyanaten und Polyphenyl-polymethylen-polyisocyanaten (roh-MDI) in mehreren Reaktionsstufen das dadurch gekennzeichnet ist, daß man

A. Anilin mit O-Alkylcarbamidsäureestern und gegebenenfalls Alkoholen oder Anilin mit O-Alkylcarbamidsäureestern, Harnstoff und Alkoholen in Gegenwart oder Abwesenheit von Katalysatoren zu N-Phenyl-urethanen umsetzt und den dabei gebildeten Ammoniak gegebenenfalls abtrennt,

B. die erhaltenen N-Phenyl-urethane mit Formaldehyd oder vorzugsweise bifunktionellen Verbindungen der Formel

$X-CH_2-X$

in der X einen RO-, RS- oder ROCO-Rest und R einen Alkylrest bedeuten, in Gegenwart einer Säure oder Carbonsäure zu Mischungen aus Methylen-bis-phenylurethanen und Polymethylen-polyphenylurethanen kondensiert und

C. die Mischungen aus Methylen-bis-phenylurethanen und Polymethylen-polyphenylurethanen durch thermische Spaltung gegebenenfalls in Gegenwart von Katalysatoren bei Temperaturen von 175 bis 600°C in Mischungen aus Diphenylmethan-diisocyanaten und Polyphenyl-polymethylen-polyisocyanaten überführt.

Das erfindungsgemäße Verfahren kann schematisch durch die Gleichungen (I) bis (IV) veranschaulicht werden.

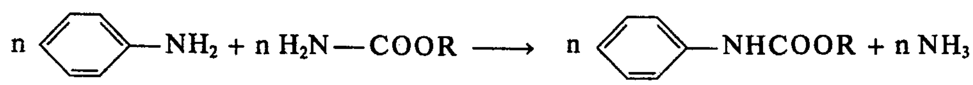

(I)

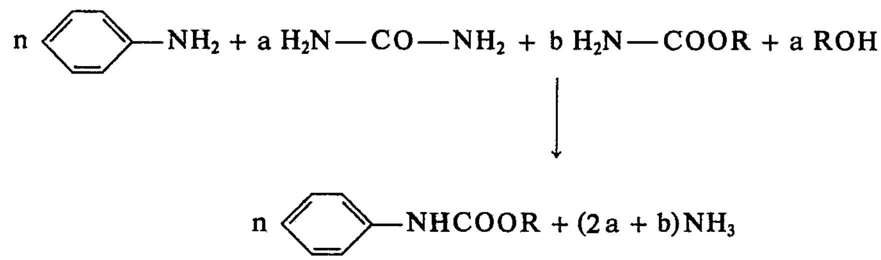

(II)

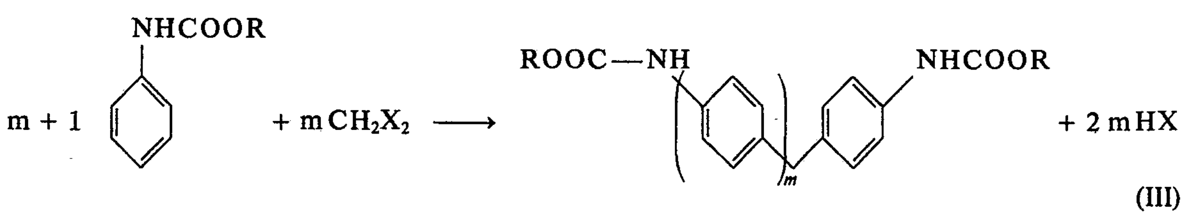

(III)

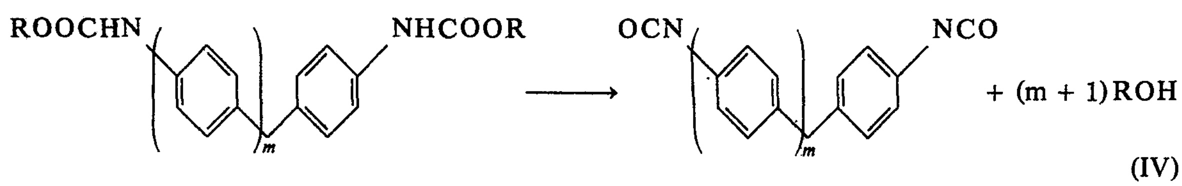

(IV)

In den Gleichungen (I), (II), (III) und (IV) bedeuten n, a, b und m ganze Zahlen, wobei nach Gleichung (II) $a+b=n$ und $a : n=1{,}5$ bis 0 und nach den Gleichungen (III) und (IV) $m=1$ bis 6 und größer sind.

Die Bildung von N-Phenyl-urethanen in einem Verfahrensschritt und in guten Ausbeuten ist überraschend. Nach bekannter Lehre werden aus Carbamidsäureestern und aromatischen Aminen N,N'-Diarylharnstoffe erhalten, die meist aus Alkohol umkristallisiert werden. Unsubstituierte Urethane reagieren in Gegenwart von aromatischen Aminen auch in Alkohol sehr leicht zu N,N'-Diarylharnstoffen weiter (Methoden der organischen Chemie, Houben—Weyl, Band 8, Seiten 152, 140 und 161, Georg Thieme Verlag, Stuttgart, 1952).

Es ist überraschend, daß N-Phenyl-urethane aus Anilin und O-Alkylcarbamidsäureestern erfindungsgemäß katalytisch bereits in Abwesenheit von Alkoholen erhalten werden, überschüssige O-Alkylcarbamidsäureester nicht zu wesentlichen Zersetzungen und Polykondensationen führen, die Reaktion durch Zusatz der genannten Katalysatoren bei relativ niederen Temperaturen technisch durchgeführt und auch in ihrer Selektivität verbessert werden kann, die Umsetzung mit Harnstoff und Alkohol

in Gegenwart mindestens eines der genannten Katalysatoren bereits bei relativ tiefen Temperaturen technisch erheblich effektiver wird, wenn sie zusammen mit größeren Mengen O-Alkylcarbamidsäureester durchgeführt wird. Hierbei ist nicht erforderlich, O-Alkylcarbamidsäureester separat herzustellen. In einer leicht praktikablen, vorzugsweise zur Anwendung kommenden Ausführungsform wird O-Alkylcarbamidsäureester zusammen mit Harnstoff und Alkohol eingesetzt und nach weitgehender bis vollständiger Umsetzung des Anilins durch Destillation abgetrennt und gegebenenfalls zurückgeführt. Das Verfahren kann auch kontinuierlich durchgeführt werden.

Überraschend ist ferner, daß die erhaltenen N-Phenyl-urethane, mit Formaldehyd oder Acetalen und Acylalen der Formeln $CH_2(OR)_2$ und $CH_2(OCOR)_2$, in Abwesenheit von Wasser und Gegenwart von Säuren oder Carbonsäuren glatt in guten Ausbeuten und mit hoher Reinheit in roh-MDU übergeführt werden können.

Zur Herstellung der N-Phenylurethane werden O-Alkylcarbamidsäureester nach dem erfindungsgemäßen Verfahren mit Anilin in Abwesenheit oder vorzugsweise in Gegenwart von Katalysatoren und gegebenenfalls Harnstoff und Alkohol zur Reaktion gebracht.

Geeignete O-Alkylcarbamidsäureester besitzen die Formeln $H_2N-COOR$, in der R einen gegebenenfalls substituierten aliphatischen, cycloaliphatischen oder aromatisch-aliphatischen Rest bedeutet. In Betracht kommen beispielsweise O-Alkylcarbamidsäureester auf Basis von primären gegebenenfalls substituierten aliphatischen Monoalkoholen mit 1 bis 20 Kohlenstoffatomen, vorzugsweise 1 bis 10 Kohlenstoffatomen, wie Carbamidsäure-methylester, -äthylester, -propylester, -n-butylester, -isobutylester, -2- und -3-methylbutylester, -neopentylester, -pentylester, -2-methyl-pentylester, -n-hexylester, -2-äthylhexylester, -heptylester, -n-octylester, -n-nonylester, -n-decylester und -n-dodecylester, -2-phenylpropylester und -benzylester, auf Basis von sekundären aliphatischen und cycloaliphatischen Monoalkoholen mit 3 bis 15 Kohlenstoffatomen, vorzugsweise 3 bis 6 Kohlenstoffatomen, wie Carbamidsäure-isopropylester, -sek.-butylester, -sek.-iso-amylester, -cyclopentylester, -cyclohexylester, -bicyclo(2,2,1)-heptylester und tert.-butyl-cyclohexylester. Vorzugsweise verwendet werden Carbamidsäure-methylester,-äthylester, -propylester, -butylester, -isobutylester, -2- und -3-methylbutylester, -pentylester, -hexylester, -2-äthylhexylester, -heptylester, -octylester und -cyclohexylester.

Als Alkohole können beliebige, gegebenenfalls substituierte primäre oder sekundäre aliphatische Alkohole sowie Mischungen davon verwendet werden. Vorzugsweise wird der den O-Alkylcarbamidsäureestern entsprechende Alkohol eingesetzt.

In Betracht kommen beispielsweise primäre aliphatische Alkohole mit 1 bis 20 Kohlenstoffatomen, vorzugsweise 1 bis 10 Kohlenstoffatomen, wie Methanol, Äthanol, Propanol, n-Butanol, n-Pentanol, Neopentylalkohol, 2-Methyl-butanol, 2-Methyl-pentanol, n-Hexanol, n-Heptanol, n-Octanol, Nonanol, n-Decanol und n-Dodecanol, sekundäre aliphatische und cycloaliphatische Alkohole mit 3 bis 15 Kohlenstoffatomen, vorzugsweise 3 bis 6 Kohlenstoffatomen, wie Isopropanol, sec.-Butanol, sec.-Isoamylalkohol, Cyclopentanol, 2-, 3- oder 4-Methyl-cyclohexanol, Cyclohexanol und Bicyclo(2,2,1)-heptanol. Vorzugsweise verwendet werden als Monoalkohole Methanol, Äthanol, Propanol, Isopropanol, n-Butanol, 2-Äthyl-butanol, 2- und 3-Methylbutanol, n-Pentanol, n-Hexanol, 2-Äthyl-hexanol, Heptanol, Octanol und Cyclohexanol.

Die Alkohole können gegebenenfalls mit anderen unter den Reaktionsbedingungen inerten organischen Lösungsmitteln gemischt werden.

Zur Herstellung der N-Phenyl-urethane in Abwesenheit von Katalysatoren werden Anilin, O-Alkylcarbamidsäureester und gegebenenfalls Alkohol im Molverhältnis 1 : 0,5 bis 20 : 1 bis 100, vorzugsweise 1 : 0,8 bis 10 : 1 bis 50 und insbesondere 1 : 1 bis 6 : 1 bis 5 zur Reaktion gebracht. Erfolgt die Umsetzung jedoch in Gegenwart von Katalysatoren, so haben sich Molverhältnisse von Anilin zu O-Alkylcarbamidsäureester zu gegebenenfalls Alkohol von 1 : 0,5 bis 20 : 0 bis 100, vorzugsweise 1 : 0,8 bis 10 : 0 bis 30 und insbesondere 1 : 1 bis 6 : 0 bis 5 besonders gut bewährt.

Wie bereits ausgeführt wurde, werden zur Herstellung der N-Phenylurethane vorzugsweise neben O-Alkylcarbamidsäureester zusätzlich Harnstoff und Alkohol mitverwendet, wobei das Verhältnis von Anilin zur Summe aus O-Alkylcarbamidsäureester und Harnstoff ebenfalls 1 : 0,5 bis 20, vorzugsweise 1 : 0,8 bis 10 und insbesondere 1 : 1 bis 6 beträgt, das Molverhältnis von Harnstoff zu Anilin gleich oder kleiner als 1,5, vorzugsweise 1,25 bis 0,75 und das Molverhältnis Harnstoff zu Alkohol gleich oder kleiner als 1 sind.

Harnstoff wird zweckmäßigerweise in handelsüblicher Form und Reinheit eingesetzt.

Zur Erhöhung der Reaktionsgeschwindigkeit und Verbesserung der Ausbeute wird die Reaktion vorzugsweise in Gegenwart eines oder mehrerer Katalysatoren durchgeführt. Als Katalysatoren eignen sich anorganische und organische Verbindungen, die ein oder mehrere Kationen, vorzugsweise ein Kation, von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14th. Edition, publiziert von Chemical Rubber Publishing Co. 2310 Superior Ave. N. E. Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Wismut, Antimon, Kupfer, Silber, Gold, Zink, Quecksilber, Cer, Titan, Vanadium, Chrom, Molybdän, Mangan, Eisen, Kobalt und Nickel. Vorzugsweise Verwendung finden die Kationen von Lithium, Calcium, Aluminium, Zinn, Wismut, Antimon, Kupfer, Zink, Titan, Vanadium, Chrom, Molybdän, Mangan, Eisen und Kobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumäthanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-isobutylat, Aluminiumtrichlorid, Wismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Kobaltacetat, Kobaltchlorid, Kobaltsulfat, Kobaltnaphthenat, Nickelchlorid, Nickelacetat und Nickelnaphthenat sowie deren Gemische.

Die Katalysatoren werden zweckmäßigerweise in Mengen, die 0,0001 bis 0,1, vorzugsweise 0,0005 bis 0,05 Äquivalenten des Metallkations, bezogen auf Anilin entsprechen, verwendet. Die Metallionen können auch gebunden an Ionenaustauscher in heterogener Phase eingesetzt werden.

Die Umsetzung wird bei erhöhten Temperaturen, beispielsweise in Abwesenheit von Katalysatoren bei Temperaturen von 160 bis 300° C, vorzugsweise 170 bis 230° C und insbesondere 175 bis 210° C und in Gegenwart von Katalysatoren bei Temperaturen von 100 bis 250° C, vorzugsweise 120 bis 210° C und insbesondere 135 bis 190° C sowie Drücken von 0,1 bis 120 bar, vorzugsweise 0,5 bis 60 bar und insbesondere 1 bis 40 bar durchgeführt, wobei es sich als vorteilhaft erwiesen hat, den entstehenden Ammoniak aus der Reaktionsmischung, beispielsweise durch Destillation, abzutrennen. Bei gegebener Temperatur wird die Umsetzung dann vorzugsweise unter einem Druck durchgeführt, bei dem der entstehende Ammoniak selektiv aus dem Reaktionsgemisch abdestilliert werden kann. Die entsprechenden Werte können Tabellen mit physikalischen Kenndaten des Ammoniaks und der Alkohole entnommen werden. Für den genannten Temperaturbereich ergeben sich Reaktionszeiten von 0,5 bis 100 Stunden, vorzugsweise von 1 bis 50 Stunden und insbesondere 2 bis 25 Stunden.

Die N-Phenylurethane werden nach der erfindungsgemäßen Methode zweckmäßigerweise wie folgt hergestellt: Anilin, O-Alkylcarbamidsäureester und gegebenenfalls Alkohole bzw. vorzugsweise Anilin, O-Alkylcarbamidsäureester, Harnstoff und Alkohole werden in den genannten Mengenverhältnissen gemischt und vorzugsweise in Gegenwart eines Katalysators in einem Reaktionsgefäß, ausgestattet mit einer Vorrichtung zur Abtrennung des Ammoniaks, gegebenenfalls unter Rühren erhitzt. Der gebildete Ammoniak kann nach beendeter Umsetzung abgetrennt werden; vorzugsweise wird er jedoch im Laufe der Reaktion kontinuierlich oder absatzweise abdestilliert. Hierbei kann es zweckmäßig sein, insbesondere bei der Umsetzung in Gegenwart von niedermolekularen Alkoholen unter Druck, den Ammoniak mit Hilfe eines unter den Reaktionsbedingungen inerten Schleppmittels, beispielsweise eines Gases, wie Stickstoff, oder zusammen mit einem Teil des Alkohols abzutrennen. Aus der erhaltenen Reaktionsmischung wird anschließend, gegebenenfalls nach Abtrennen des Katalysators und Abfiltrieren von Feststoffen das N-Phenylurethan isoliert, beispielsweise durch fraktionierte Destillation, durch Abdestillieren des überschüssigen O-Alkylcarbamidsäureesters und/oder Alkohols, durch partielles Abdestillieren des überschüssigen O-Alkylcarbamidsäureesters und/oder Alkohols und Auskristallisieren des N-Phenylurethans, durch Auskristallisieren, durch Ausfällen mit oder auch durch Umkristallisieren aus anderen Lösungsmitteln. Die Katalysatoren können gegebenenfalls abgetrennt werden, z. B. durch Sedimentieren, Abfiltrieren, Auswaschen oder Binden mit Ionenaustauscher.

Die erfindungsgemäß hergestellten N-Phenylurethane werden mit Formaldehyd, formaldehydabspaltenden Verbindungen oder vorzugsweise Verbindungen der Formel $X-CH_2-X$, wobei X einen RO-, RS- oder ROCO-Rest und R einen Alkylrest bedeuten, in Gegenwart von Säuren oder Carbonsäuren zu Mischungen aus Methylen-bis-phenylurethanen und Polymethylen-polyphenylurethanen (roh-MDU) kondensiert.

Die Herstellung des roh-MDU kann beispielsweise gemäß dem Stand der Technik zweistufig erfolgen durch Kondensation von N-Phenylurethan und Formaldehyd nach DE-PS 1 042 891 und Vervollständigung der Umlagerung nach DE-OS 2 832 379.

Vorteilhaft wird die Kondensation von N-Phenylurethan erfindungsgemäß jedoch einstufig mit Formaldehyd oder formaldehydabspaltenden Verbindungen, z. B. Trioxan oder Paraformaldehyd in einer gegebenenfalls substituierten Carbonsäure, z. B. Propionsäure, Chloressigsäure oder Essigsäure, vorzugsweise einer Carbonsäure deren pks-Wert kleiner als 4 ist, z. B. Ameisensäure, Oxalsäure, Dichloressigsäure, Trichloressigsäure oder Trifluoressigsäure als Lösungsmittel (nach deutsche Patentanmeldung, P 2 942 137.2 der BASF Aktiengesellschaft) oder insbesondere mit bifunktionellen Verbindungen der Formeln $CH_2(SR)_2$, $CH_2(OR)_2$ oder $CH_2(OCOR)_2$, in denen R einen Alkylrest mit 1 bis 6, vorzugsweise 1 bis 3 C-Atomen, bedeuten, in Gegenwart einer starken Säure durchgeführt. Die

Thioacetale, Acetale oder Acylale bilden unter den Umsetzungsbedingungen praktisch keinen freien Formaldehyd und schließen die Bildung von Reaktionswasser aus. Die Verwendung der Acetale und Acylale ist von besonderem technischem Interesse. Vorzugsweise verwendet man solche Verbindungen der genannten Art, in denen der Alkylrest mit dem Alkohol übereinstimmt, der dem N-Phenylurethan zugrunde liegt. Beispielhaft genannt seien Formaldehyddimethylacetal (= Dimethylformal), Diäthylformal und Diacetoxymethan. Die Verwendung von Dimethylformal ist besonders zweckmäßig, da es sich leicht und wirtschaftlich aus wäßrigem Formaldehyd und Methanol gewinnen läßt.

Die Kondensation der N-Phenylurethane mit Formaldehyd in Carbonsäuren oder mit den bifunktionellen Verbindungen in Gegenwart einer Säure wird bei Temperaturen von 50 bis 160° C, vorzugsweise 90 bis 140° C, insbesondere 90 bis 120° C durchgeführt.

Das Molverhältnis Formaldehyd bzw. bifunktionelle Verbindung zu N-Phenylurethan liegt allgemein bei 1 : 0,5 bis 1 : 10, vorzugsweise bei 1 : 1,5 bis 1 : 3. Will man hauptsächlich Methylen-bis-phenylurethan unter weitgehender Vermeidung der Bildung von Methylen-polyphenylurethanen herstellen, so verwendet man vorzugsweise ein Verhältnis von 1 : 4 bis 1 : 8. Sofern gegebenenfalls substituierte Carbonsäuren als Lösungsmittel verwendet werden, werden diese in einer Menge von 50 bis 500 g, vorzugsweise 100 bis 200 g pro Mol N-Phenylurethan eingesetzt.

Als Säuren, die man z. B. in Mengen von 1 bis 100, vorzugsweise 20 bis 60 Mol.%, bezogen auf N-Phenylurethan, anwendet, sind z. B. Phosphorsäure, Schwefelsäure, Chlorwasserstoff, Alkylsulfonsäure wie Methansulfonsäure und Trifluormethansulfonsäure oder Arylsulfonsäure wie p-Toluolsulfonsäure geeignet. Entsprechend einer vorteilhaften Ausführungsform verwendet man eine starke Säure, die vom Reaktionsgemisch destillativ abgetrennt werden kann, beispielsweise Methansulfonsäure und Trifluormethansulfonsäure. Auf diese Weise entfällt die Aufarbeitung des Reaktionsgemisches mit Wasser oder Basen und die Säure kann direkt erneut der Reaktion zugeführt werden.

Nach einer weiteren vorteilhaften Ausführungsform verwendet man als Säuren stark saure organische Kationenaustauscher, z. B. sulfonsaure Austauscherharze. Beispielhaft genannt seien: ®Lewasorb AC-10 (Bayer AG), ®Lewatit SPC-108 (Bayer AG), ®Amberlyst-15 (Rohm and Haas, Co.) oder Nafion-Säure (Dupont de Nemours). Diese Ionenaustauscher werden nach an sich bekannten Methoden entweder im Reaktionsgemisch suspendiert oder in einem Festbett angeordnet.

Man führt die Kondensation vorzugsweise in Abwesenheit von Wasser, d. h. unter Verwendung von Säuren durch, die praktisch kein Wasser enthalten. Man kann sowohl ohne als auch in Gegenwart eines nicht wäßrigen Lösungsmittels, beispielsweise Benzol, Methylcyclohexan, Essigsäure, Sulfolan, Methanol, Methylacetat, Nitrobenzol, Chlorbenzol, Dichlorbenzol oder chlorierte aliphatische Kohlenwasserstoffe arbeiten.

Die Umsetzung, die etwa nach 0,5 bis 20 Stunden beendet ist, wird im allgemeinen so durchgeführt, daß man entweder zu einem Gemsich aus dem N-Phenylurethan und der Säure und/oder Carbonsäure unter Rühren bei der Reaktionstemperatur beispielsweise ein Gemisch aus Paraformaldehyd und Carbonsäure oder die bifunktionelle Verbindung langsam zugibt oder daß man ein Gemisch aus dem N-Phenylurethan, Paraformaldehyd und Carbonsäure bzw. der bifunktionellen Verbindung und der Säure unter Rühren erhitzt und die Gemische die entsprechende Zeit auf der Reaktionstemperatur hält. Die Isolierung des Reaktionsproduktes erfolgt nach herkömmlichen Methoden, z. B. durch destillative Abtrennung des Lösungsmittels und Extraktion des Katalysators mit Wasser oder Neutralisation mit einer Base. Gegebenenfalls vorhandene nichtumgesetzte Ausgangsprodukte können mittels einer Vakuumdestillation abgetrennt werden.

Die Kondensation der N-Phenylurethane mit Formaldehyd, formaldehydabspaltenden Verbindungen bzw. den genannten Formaldehydderivaten kann in Einzelansätzen oder als kontinuierliches Verfahren durchgeführt werden.

Nach dem erfindungsgemäßen Kondensationsverfahren werden roh-MDU erhalten, die je nach gewählten Molverhältnissen von N-Phenylurethan und Formaldehyd bzw. bifunktioneller Verbindung Methylen-bis-phenylurethane in Mengen von 20 bis 90 Gew.-%, vorzugsweise von 40 bis 80 Gew.-%, bezogen auf das Gesamtgewicht besitzen und folgende Isomerenverteilung aufweisen: Methylen-bis-(4-phenylurethan) 70 bis 95 Gew.-%, vorzugsweise 80 bis 95 Gew.-% und Methylen-(2-phenylurethan)-4-phenylurethan 30 bis 5 Gew.-%, vorzugsweise 5 bis 20 Gew.-%, jeweils bezogen auf das Gesamtgewicht an Methylen-bis-phenylurethanen.

Gegebenenfalls kann es zweckmäßig sein, aus dem roh-MDU die Methylen-bis-phenylurethane ganz oder vorzugsweise teilweise abzutrennen und gegebenenfalls in die Isomeren zu trennen. Dies kann beispielsweise durch fraktionierte Kristallisation erfolgen.

Die erfindungsgemäß hergestellten roh-MDU können in an sich bekannter Weise bei Temperaturen von 175 bis 600° C in Abwesenheit von Katalysatoren, beispielsweise gemäß DE-OS 2 410 505 (US 3 870 739) oder in Gegenwart von Katalysatoren beispielsweise analog den Angaben der DE-OS 1 944 719 (GB-PS 1 247 451) in die entsprechenden roh-MDI thermisch gespalten werden. Vorzugsweise wird die thermische Spaltung in flüssiger Phase in Gegenwart von Lösungsmitteln katalysatorfrei, beispielsweise analog den Angaben der DE-AS 2 421 503 (US 3 962 302) oder DE-AS 2 530 001 (US 3 919 280 und US 3 919 279) oder insbesondere in Gegenwart von Lösungsmitteln und gelösten Katalysatoren, beispielsweise analog den Angaben der DE-OS 2 635 490 bei Temperaturen von 175 bis 350° C durchgeführt.

Da die Entfernung gelöster Katalysatoren aus den Reaktionsgemischen problematisch ist, werden erfindungsgemäß von den bekannten Verfahren bevorzugt solche empfohlen, die unkatalysierte Spaltungen beschreiben. Es hat sich aber überraschend als vorteilhaft erwiesen, die thermische Spaltung durch Heterogenkatalyse mit Metallen aus der Reihe Zink, Aluminium, Titan, Eisen, Chrom, Kobalt und Nickel, wobei bevorzugt Zink und Aluminium eingesetzt werden, bzw. diese, gegebenenfalls in Kombination mit anderen Metallen, z. B. Vanadium und Wolfram, enthaltende Legierungen in oberflächenreicher Form, z. B. in Form von Metallpulvern, -granulaten mit durchschnittlichen Durchmessern von 2 bis 10 mm, -spänen oder -wolle, zu beschleunigen. Die Metalle sind nicht nur gute Wärmeüberträger, sondern sie haben darüber hinaus einen guten, technisch vorteilhaft verwertbaren Katalyseeffekt und ermöglichen deutliche Senkungen der Reaktionstemperaturen und -zeiten, so daß Nebenreaktionen, wie Polymerisationen eine geringere Rolle spielen.

Die Katalysatoren können in verschiedenen Anordnungen angewandt werden, beispielsweise als Festbett, z. B. durch Beschicken von Rohr- oder Kesselreaktoren mit Metallgranulaten, -ringen, -spänen oder -wolle, so daß das Reaktionsgemisch kontinuierlich durch das Katalysator-Festbett geleitet werden kann, oder auch in Suspension in Rührreaktoren.

Als Lösungsmittel werden bei der erfindungsgemäß bevorzugten Thermolyse in flüssiger Phase solche verwendet, die gegenüber den Isocyanaten und übrigen Komponenten unter den Reaktionsbedingungen inert sind und deren Siedepunkt unterhalb dem Siedepunkt von 4,4′-Diphenylmethan-diisocyanat und vorzugsweise höher als der des abzuspaltenden Alkohols liegt. Ferner darf die kritische Temperatur des Lösungsmittels nicht unterhalb 175°C liegen. Vorteilhaft ist außerdem, wenn das roh-MDU in dem Lösungsmittel löslich ist, obgleich dies nicht unbedingt notwendig ist. Beispielhaft genannt seien: aliphatische Kohlenwasserstoffe, wie die höheren Alkane, Decan, Undecan, Dodecan, Tridecan, Tetradecan, Pentadecan, Hexadecan,Heptadecan und Dekalin, gegebenenfalls substituierte aromatische Kohlenwasserstoffe, wie z. B. Naphthalin, 1- und 2-Methylnaphthalin, 1,2-, 1,4-, 1,6-, 2,7-, 2,6- und 2,3-Dimethylnaphthalin, 1-Äthylnaphthalin, Toluol, 1,2-, 1,3- und 1,4-Dimethylbenzol, 1,2,4- und 1,3,5-Trimethylbenzol, 1,2,3,5- und 1,2,4,5-Tetramethylbenzol, 1,3,5-Triäthylbenzol, Hexyl-, Heptyl-, Octyl-, Nonyl-, Decyl- und Dodecylbenzol, Hexamethylbenzol, Hexaäthylbenzol, Diphenyl, 4,4′-Dimethyldiphenyl, Dibenzyl, Diphenylmethan und 4,4′-Dimethyl-diphenylmethan, halogensubstituierte aromatische Kohlenwasserstoffe, wie z. B. Chlorbenzol, 1,2- und 1,4-Dichlorbenzol, 1,4-Dijodbenzol, 1,2,3- und 1,3,5-Trichlorbenzol, 1,2,3,4-, 1,2,3,5- und 1,2,4,5-Tetrachlorbenzol, Pentachlorbenzol, 1- und 2-Fluornaphthalin, 1- und 2-Chlornaphthalin, 1- und 2-Jodnaphthalin und Diphenyl-dichlormethan; Nitrogruppenhaltige aromatische Kohlenwasserstoffe, wie z. B. Nitrobenzol, 3-Nitrotoluol, 2-Nitro-m-xylol, 5-Nitro-m-xylol und 4-Nitroanisol, aliphatische und aromatische Ketone, wie z. B. Cyclohexanon, Cycloheptanon, Di-n-butylketon, Di-n-amylketon, $\alpha$-Tetralon, Acetophenon, Propiophenon, Benzophenon, 3-Methylbenzophenon, Dodecanon-2 und Tridecanon-2, Sulfone und Carbonsäureester, wie z. B. Sulfolan, Diäthylsulfon, Phthalsäuredimethylester, Phthalsäurediäthylester, Benzoesäurepropylester und Laurinsäureäthylester und Äther, wie z. B. Diäthylenglykol-dimethyläther, Diäthylenglykol-diäthyläther, Diisoamyläther, Di-n-amyläther, Resorcindimethyläther, Resorcindiäthyläther, Phenyloctyläther, Phenylbenzyläther, Dibenzyläther, Diphenyläther, $\alpha$-Methylnaphthyläther und $\beta$-Äthylnaphthyläther.

Die thermische Spaltung von roh-MDU in Gegenwart von Metallen wird bei Temperaturen von 175 bis 600°C, vorzugsweise in einem Lösungsmittel bei 175 bis 350°C, insbesondere bei 230 bis 350°C unter vermindertem, normalem oder erhöhtem Druck diskontinuierlich oder vorzugsweise kontinuierlich durchgeführt. Die Spaltung und Trennung der Produkte können nacheinander oder vorzugsweise gleichzeitig erfolgen. Man kann das roh-MDU, z. B. als Pulver geschmolzen, oder als Suspension bzw. Lösung in einem inerten Lösungsmittel dem Reaktor zuführen, der wahlweise auf eine bestimmte Temperatur und einen bestimmten Druck gehalten wird. Beispielsweise kann man die Lösung von roh-MDU durch eine Kaskade leiten, die abwechselnd aus Rohrspaltreaktoren und Trennkolonnen besteht. In bevorzugten Ausführungsformen leitet man die Lösung des roh-MDU, entsprechend 0,1 bis 5, vorzugsweise 0,2 bis 3 Äquivalenten Urethan je Liter und Stunde kontinuierlich in einen Reaktor bzw. eine Reaktorkaskade unter simultaner Spaltung und Abtrennung des Alkohols über eine oder mehrere Trennkolonnen, wobei man das Temperatur/Druckverhältnis zweckmäßig so wählt, daß das Lösungsmittel unter Rückfluß gehalten wird oder teilweise zusammen mit dem Alkohol abdestilliert. Schließlich kann man das Lösungsmittel durch schonende Destillation, gegebenenfalls unter Strippen, vorzugsweise über oberflächenreiche Verdampfer mit kurzer Verweilzeit, wobei Diphenylmethan-diisocyanate als Trägermaterial fungieren, abdestillieren und roh-MDI als Bodenfraktion abziehen. Hierbei hat sich als vorteilhaft gezeigt, einen Anteil an Diphenylmethan-diisocyanat mit dem Lösungsmittel abzudestillieren und mit diesem zusammen in den Kreislauf zurückzuführen.

Die in den Beispielen genannten Teile beziehen sich auf das Gewicht.

Beispiel 1

93 Teile Anilin werden mit 450 Teilen Carbamidsäuremethylester, 0,9 Teilen Eisen-(II)-acetat und 95 Teilen Methanol 6 Stunden lang auf 175—180°C erhitzt, wobei über ein Druckventil im Reaktionsgefäß

ein Druck von 5 bis 6 bar eingestellt wird. Der während der Reaktion gebildete Ammoniak wird unter Verwendung von 15 l Stickstoff je l Reaktionsgemisch und Stunde als Schleppmittel kontinuierlich abdestilliert. Nach beendeter Reaktion destilliert man nicht umgesetztes Anilin, überschüssiges Methanol und überschüssigen Carbamidsäuremethylester bei ca. 20 mbar ab und erhält durch Destillation bei 110 bis 113°C und 0,1 mbar 127 Teile N-Phenylmethylurethan (88,5% der Theorie, bezogen auf umgesetztes Anilin). Im Destillationsrückstand befindet sich weiteres N-Phenyl-methylurethan. Der Umsatz an Anilin beträgt 95% (gaschromatographisch ermittelt).

Das so erhaltene N-Phenylmethylurethan wird in einem Rührautoklaven zusammen mit 32 Teilen Dimethylformal, 93 Teilen Nitrobenzol und 17 Teilen Schwefelsäure unter Rühren auf 100°C erhitzt und 10 Stunden lang bei dieser Temperatur gerührt. Nach Beendigung der Umsetzung und Extraktion der Säure mit Wasser werden unter vermindertem Druck das Lösungsmittel und nicht umgesetztes Ausgangsprodukt abdestilliert. Man erhält 126 Teile eines Destillationsrückstandes, der entsprechend der Hochdruck-Flüssigchromatographie-Analyse aus 52% Di-(methoxicarbonylamino)-diphenylmethan, 30% 3-Kern und 18% höherkernigen Polymethylen-polyphenylurethanen (roh-MDU) besteht.

126 g des so erhaltenen roh-MDU werden in 550 g Sulfolan gelöst und mit einer Zulaufrate von 300 g pro Liter Reaktionsraum und Stunde in einen mit Zinkspänen gefüllten, auf 300°C erhitzen Rohrreaktor aus Quarzglas eingepumpt. Das bei der Spaltung gebildete Methanol wird gasförmig abgetrennt und in einer mit Trockeneis gekühlten Vorlage kondensiert. Man erhält 658 g Reaktoraustrag, aus dem bei 90 bis 95°C und 0,1 mbar das Spaltlösungsmittel Sulfolan abdestilliert wird. Es verbleiben 112 g eines Gemisches von 57% Diphenylmethan-diisocyanaten, 31% 3-Kern und 12% höherkernigen Polyphenylpolymethylenpolyisocyanaten.

## Beispiel 2

60 Teile Anilin werden mit 172 Teilen Carbamidsäureäthylester, 38 Teilen Harnstoff, 0,75 Teilen Kobaltacetat und 90 Teilen Äthanol 5 Stunden lang auf 175 bis 180°C erhitzt, wobei über ein Druckventil im Reaktionsgefäß ein Druck von 5 bis 7 bar eingestellt wird. Der während der Reaktion gebildete Ammoniak wird unter Verwendung von 12 l Stickstoff je l Reaktionsgemisch und Stunde als Schleppmittel kontinuierlich abdestilliert. Nach beendeter Reaktion destilliert man nicht umgesetztes Anilin, überschüssiges Äthanol und überschüssigen Carbamidsäureäthylester bei ca. 12 mbar ab und erhält durch Destillation bei 123 bis 126°C und 0,1 mbar 89 Teile N-Phenyläthylurethan (86,2% der Theorie, bezogen auf umgesetztes Anilin). Im Destillationsrückstand befindet sich noch weiteres Phenyläthylurethan. Der Umsatz an Anilin beträgt 97% (gaschromatographisch ermittelt).

Das so erhaltene N-Phenyläthylurethan wird in einem Rührreaktor mit 9 Teilen Paraformaldehyd, 60 Teilen Essigsäure und 18 Teilen Lewatit SPC-108 unter Rühren auf 100°C erhitzt und 10 Stunden lang bei dieser Temperatur gerührt. Nach Beendigung der Umsetzung werden der Katalysator abfiltriert und das Lösungsmittel und nicht umgesetztes Ausgangsmaterial unter vermindertem Druck abdestilliert. Man erhält 88 Teile eines Destillationsrückstandes, der entsprechend der Hochdruck-Flüssigchromatographie-Analyse aus 55% Di-(äthoxicarbonylamino)-diphenylmethan, 29% 3-Kern und 16% höherkernigen Polymethylen-polyphenylurethanen (roh-MDU) besteht.

88 g des so erhaltenen roh-MDU werden in 500 g Decylbenzol gelöst und mit einer Zulaufrate von 300 g pro Liter Reaktionsraum und Stunde in einen mit Aluminiumgranulat gefüllten, auf 320°C erhitzten Rohrreaktor aus Quarzglas eingepumpt. Das bei der Spaltung gebildete Äthanol wird gasförmig abgetrennt und in einer mit Trockeneis gekühlten Vorlage kondensiert.

Man erhält 545 g Reaktoraustrag, aus dem bei 85 bis 95°C und 0,2 mbar das Spaltlösungsmittel Decylbenzol abdestilliert wird. Es verbleiben 69 g eines Gemisches von 58% Diphenylmethan-diisocyanaten, 29% 3-Kern und 13% höherkernigen Polyphenyl-polymethylen-polyisocyanten.

## Beispiel 3

93 Teile Anilin werden mit 450 Teilen Carbamidsäuremethylester, 0,9 Teilen Eisen-(II)-acetat und 95 Teilen Methanol 6 Stunden lang auf 175 bis 180°C erhitzt, wobei über ein Druckregelventil im Reaktionsgefäß ein Druck von 5 bis 7 bar eingestellt wird. Der während der Reaktion gebildete Ammoniak wird unter Verwendung von 15 l Stickstoff je 1 Reaktionsgemisch und Stunde als Schleppmittel kontinuierlich abdestilliert. Nach beendeter Reaktion destilliert man nicht umgesetztes Anilin, überschüssiges Methanol und überschüssigen Carbamidsäuremethylester bei ca. 20 mbar ab und erhält durch Destillation bei 109 bis 113°C und 0,1 mbar 133 Teile N-Phenylmethylurethan (93,7% der Theorie, bezogen auf umgesetztes Anilin). Im Destillationsrückstand befindet sich noch weiteres Phenylmethylurethan. Der Umsatz an Anilin beträgt 94%.

Das so erhaltene Phenylmethylurethan wird in einem Rührreaktor zusammen mit 58 Teilen Diacetoximethan, 106 Teilen Nitrobenzol und 44 Teilen Lewatit SPC-108 unter Rühren auf 100°C erhitzt und 5 Stunden bei dieser Temperatur gerührt. Nach Beendigung der Umsetzung werden der Katalysator abgetrennt und unter vermindertem Druck Nitrobenzol und nicht umgesetztes Ausgangsmaterial abdestilliert. Man erhält 125 Teile eines Destillationsrückstands, der entsprechend der Analyse durch Hochdruck-Flüssigchromatographie aus 76% Di-(methoxicarbonylamino)-diphenylmethan, 16% 3-Kern und 8% höherkernigen Polymethylen-polyphenylurethanen (roh-MDU) besteht.

125 g des so erhaltenen roh-MDU werden in 375 g Decylbenzol gelöst und mit einer Zulaufrate von 320 g pro Liter Reaktionsgemisch und Stunde in einen mit Zinkspänen gefüllten, auf 310°C, erhitzten Rohrreaktor aus Quarzglas eingepumpt. Das bei der Spaltung gebildete Methanol wird gasförmig abgetrennt und in einer mit Trockeneis gekühlten Vorlage kondensiert. Man erhält 472 g Reaktoraustrag, aus dem bei 85 bis 90°C und 0,1 bis 0,2 mbar das Spaltlösungsmittel Decylbenzol abdestilliert wird. Es verbleiben 102 g eines Gemisches von 78% Diphenylmethan-diisocyanaten, 17% 3-Kern und 5% höherkernigen Polyphenyl-polymethylen-polyisocyanaten.

**Patentansprüche**

1. Verfahren zur Herstellung von Mischungen aus Diphenylmethan-diisocyanaten und Polyphenyl-polymethylen-polyisocyanaten, dadurch gekennzeichnet, daß man

A. Anilin mit O-Alkylcarbamidsäureestern und gegebenenfalls Alkoholen oder Anilin mit O-Alkylcarbamidsäureestern, Harnstoff und Alkoholen in Gegenwart oder Abwesenheit von Katalysatoren zu N-Phenylurethanen umsetzt und den dabei gebildeten Ammoniak gegebenenfalls abtrennt,

B. die erhaltenen N-Phenylurethane mit Formaldehyd oder bifunktionellen Verbindungen der Formel

$$X{-}CH_2{-}X$$

in der X einen RO-, RS- oder ROCO-Rest und R einen Alkylrest bedeuten, in Gegenwart einer Säure oder Carbonsäure zu Mischungen aus Methylen-bis-phenylurethanen und Polymethylen-polyphenylurethanen kondensiert und

C. die Mischungen aus Methylen-bis-phenylurethanen und Polymethylen-polyphenylurethanen durch thermische Spaltung gegebenenfalls in Gegenwart von Katalysatoren bei Temperaturen von 175°C bis 600°C in Mischungen aus Diphenylmethan-diisocyanaten und Polyphenyl-polymethylen-polyisocyanaten überführt.

2. Verfahren nach Anspruch 1, dadurch gekennzeichnet, daß man bei der Herstellung der N-Phenylurethane nach Stufe A in Gegenwart mindestens einer Verbindung, die ein oder mehrere Kationen von Metallen aus den Gruppen IA, IB, IIA, IIB, IIIA, IIIB, IVA, IVB, VA, VB, VIB, VIIB und VIIIB des Periodensystems besitzt, als Katalysator arbeitet.

3. Verfahren nach Ansprüchen 1 bis 2, dadurch gekennzeichnet, daß man zur Herstellung der N-Phenylurethane (A) die Ausgangskomponenten in solchen Mengen zur Reaktion bringt, daß das Molverhältnis von Anilin zu O-Alkylcarbamidsäureester zu Alkohol 1 : 0,5 bis 20 : 0 bis 100 beträgt.

4. Verfahren nach Ansprüchen 1 bis 3, dadurch gekennzeichnet, daß man zur Herstellung der N-Phenylurethane (A) neben O-Alkylcarbamidsäureestern zusätzlich maximal 1,5 Äquivalente Harnstoff, bezogen auf Anilin, und Alkohol mitverwendet, wobei das Verhältnis von Harnstoff zu Alkohol gleich oder kleiner als 1 ist.

5. Verfahren nach Anspruch 1, dadurch gekennzeichnet, daß man die N-Phenylurethane einstufig mit Formaldehyd, formaldehydabspaltenden Verbindungen, Acetalen der Formel $CH_2(OR)_2$ oder Acylalen der Formel $CH_2(OCOR)_2$, in denen R einen Alkylrest bedeutet, in Gegenwart von Säuren oder Carbonsäuren mit pks-Werten kleiner als 4 bei Temperaturen von 50 bis 150°C zu Mischungen aus Methylen-bis-phenylurethanen und Polymethylen-polyphenylurethanen (B) kondensiert.

6. Verfahren nach Ansprüchen 1 und 5, dadurch gekennzeichnet, daß man zur Kondensation (B) der N-Phenylurethane als Säuren Schwefelsäure, Methansulfonsäure, Trifluormethansulfonsäure oder stark saure organische Kationenaustauscher und als Carbonsäuren Ameisensäure, Oxalsäure, Dichloressigsäure, Trichloressigsäure oder Trifluoressigsäure verwendet.

7. Verfahren nach Anspruch 1, dadurch gekennzeichnet, daß man die Mischungen aus Methylen-bis-phenylurethanen und Polymethylen-polyphenylurethanen in flüssiger Phase in einem Lösungsmittel bei Temperaturen von 175 bis 350°C in Gegenwart von in heterogener Phase vorliegenden Metallen aus der Reihe Zink, Aluminium, Titan, Eisen, Chrom, Kobalt und Nickel als Katalysatoren thermisch spaltet.

8. Verfahren nach Anspruch 7, dadurch gekennzeichnet, daß man zur thermischen Spaltung in flüssiger Phase ein Lösungsmittel verwendet, dessen Siedepunkt zwischen dem des 4,4'-Diphenylmethan-diisocyanats und dem des abgespaltenen Alkohols liegt.

9. Verfahren nach Anspruch 1 und 7, dadurch gekennzeichnet, daß man als Katalysator Aluminium oder Zink verwendet, das in der heterogenen Phase in einer oberflächenreichen Form vorliegt.

10. Verfahren nach Anspruch 1 und 7, dadurch gekennzeichnet, daß man die Mischung aus Methylen-bis-phenylurethanen und Polymethylen-polyphenylurethanen in flüssiger Phase thermisch spaltet, einen Teil des erhaltenen Diphenylmethan-diisocyanats mit dem Lösungsmittel abdestilliert und mit

diesem zusammen im Kreislauf in den Spaltreaktor zurückführt und die Mischung aus dem restlichen Teil der Diphenylmethan-diisocyanate und Polyphenyl-polymethylen-polyisocyanate als Bodenfraktion abzieht.

## Claims

1. A process for the production of a mixture of diphenylmethane diisocyanates and polyphenyl polymethylene polyisocyanates, wherein

A. aniline is reacted with an O-alkylcarbamate and, if desired, an alcohol, or with an O-alkylcarbamate, urea and an alcohol, in the presence or absence of a catalyst, to give an N-phenylurethane, and the ammonia formed in the reaction can, if desired, be separated off,

B. the resulting N-phenylurethane is condensed with formaldehyde or a bifunctional compound of the formula

$$X-CH_2-X,$$

where X is an RO-, RS- or ROCO-radical, R being alkyl, in the presence of an acid or carboxylic acid to give a mixture of methylene bis(phenylurethanes) and polymethylene polyphenylurethanes, and

C. the mixture of methylene bis(phenylurethanes) and polymethylene polyphenylurethanes is converted, by thermal cleavage at a temperature of from 175 to 600°C in the presence or absence of a catalyst, into a mixture of diphenylmethane diisocyanates and polyphenyl polymethylene polyisocyanates.

2. A process as claimed in claim 1, wherein the production of the N-phenylurethane according to step A is carried out in the presence of at least one compound containing one or more cations of metals selected from groups IA, IB, IIA, IIB, IIIA, IIIB, IVA, IVB, VA, VB, VIIB and VIIIB of the periodic table, as catalyst.

3. A process as claimed in claims 1 and 2, wherein, to produce the N-phenylurethane (A), the starting components are reacted in such amounts that the molar ratio of aniline to O-alkylcarbamate to alcohol is 1 : 0.5—20 : 0—100.

4. A process as claimed in claims 1 to 3, wherein, to produce the N-phenylurethane (A), a maximum of 1.5 equivalents of urea, based on aniline, and alcohol are used, the ratio of urea to alcohol being equal to or less than 1.

5. A process as claimed in claim 1, wherein the N-phenylurethane is condensed in a single stage with formaldehyde, a formaldehyde-eliminating compound, an acetal of the formula $CH_2(OR)_2$ or an acylal of the formula $CH_2(OCOR)_2$, in which formulae R is alkyl, in the presence of an acid or carboxylic acid having a $pk_a$ of less than 4, at a temperature of from 50 to 150°C, to give a mixture of methylene bis(phenylurethanes) and polymethylene polyphenylurethanes (B).

6. A process as claimed in claims 1 and 5, wherein sulfuric acid, methanesulfonic acid, trifluoromethanesulfonic acid or a strongly acidic organic cation exchanger is used as the acid and formic acid, oxalic acid, dichloroacetic acid, trichloroacetic acid or trifluoroacetic acid is used as the carboxylic acid, for the condensation (B) of the N-phenylurethane.

7. A process as claimed in claim 1, wherein the mixture of methylene bis(phenylurethanes) and polymethylene polyphenylurethanes is thermally cleaved in the liquid phase in a solvent at a temperature of from 175 to 350°C in the presence of a metal, which is selected from the group consisting of zinc, aluminum, titanium, iron, chromium, cobalt and nickel, and is present in the heterogeneous phase, as catalyst.

8. A process as claimed in claim 7, wherein a solvent is used for the thermal cleavage in the liquid phase, the boiling point of which is between that of the 4,4'-diphenylmethane diisocyanate and that of the eliminated alcohol.

9. A process as claimed in claims 1 and 7, wherein aluminum or zinc which is present in the heterogeneous phase and has a large surface area is used as catalyst.

10. A process as claimed in claims 1 and 7, wherein the mixture of methylene bis(phenylurethanes) and polymethylene polyphenylurethanes is thermally cleaved in the liquid phase, a portion of the resulting diphenylmethane diisocyanates is distilled off together with the solvent and is recycled together with the solvent to the cleavage reactor, and the mixture of the remaining portion of the diphenylmethane diisocyanates and polyphenyl polymethylene polyisocyanates is removed as a bottoms fraction.

**Revendications**

1. Procédé de préparation de mélanges de diphénylméthane-diisocyanates et polyphényl-polyméthylène-polyisocyanates, caractérisé paar le fait que:

A. on fait réagir de l'aniline avec des O-alkylcarbamates et éventuellement des alcools ou de l'aniline avec des O-alkylcarbamates, de l'urée et des alcools, en présence ou en l'absence de catalyseurs, pour obtenir les N-phényluréthanes et l'on sépare éventuellement l'ammoniac qui s'est formé,
B. on condense les N-phényluréthanes obtenus avec du formaldéhyde ou des composés bifonctionnels de formule

$X-CH_2-X$

dans laquelle X représente un reste RO, RS, ou ROCO et R un reste alkyle, en présence d'un acide ou d'un acide carboxylique, pour obtenir des mélanges de méthylène-bis-phényluréthanes et polyméthylène-polyphényluréthanes et
C. on transforme les mélanges de méthylène-bis-phényluréthanes et polyméthylène-polyphényluréthanes en mélange de diphénylméthane-diisocyanates et polyphényl-polyméthylène-polyisocyanates, par scission thermique, éventuellement en présence de catalyseurs, à des températures de 175° C à 600° C.

2. Procédé selon la revendication 1, caractérisé par le fait que, lors de la préparation des N-phényluréthanes selon l'étape A, on opère, comme catalyseur, en présence d'au moins un composé qui possède un ou plusieurs cations de métaux des groupes IA, IB, IIA, IIB, IIIA, IIIB, IVA, IVB, VA, VB, VIB, VIIB, et VIIIB du système périodique.

3. Procédé selon les revendications 1 à 2, caractérisé par le fait que, pour la préparation des N-phényluréthanes (A), on met en réaction les composants de départ en des quantités telles que les rapports molaires entre l'aniline, le O-alkylcarbamate et l'alcool soient 1/0,5 à 20/0 à 100.

4. Procédé selon les revendications 1 à 3, caractérisé par le fait que, pour la préparation des N-phényluréthanes (A), on utilise, à côté des O-alkylcarbamates, additionnellement, au maximum 1,5 équivalent d'urée, rapportée à l'aniline, et de l'alcool, le rapport entre l'urée et l'alcool étant égal ou inférieur à 1.

5. Procédé selon la revendication 1, caractérisé par le fait que l'on condense les N-phényluréthanes, en un seul temps, avec du formaldéhyde, des composés éliminant du formaldéhyde, des acétals de formule $CH_2(OR)_2$ ou des acylals de formule $CH_2(OCOR)_2$, dans lesquels R représente un reste alkyle, en présence d'acétates ou d'acides carboxyliques de pks inférieurs à 4, à des températures de 50 à 150° C, pour obtenir des mélanges de méthylène-bis-phényluréthanes et polyméthylène-polyphényluréthanes (B).

6. Procédé selon les revendications 1 et 5, caractérisé par le fait que, par la condensation (B) des phényluréthanes, on utilise, comme acides, l'acide sulfurique, l'acide méthanesulfonique, l'acide trifluorométhanesulfonique ou des échangeurs de cations organiques fortement acides et, comme acides carboxyliques, l'acide formique, l'acide oxalique, l'acide dichloracétique, l'acide trichloracétique ou l'acide trifluoracétique.

7. Procédé selon la revendication 1, caractérisé par le fait que l'on scinde thermiquement les mélanges de méthylène-bis-phényluréthanes et polyméthylène-polyphényluréthanes, en phase liquide, dans un solvant, à des températures de 175 à 350° C, en présence, comme catalyseurs, de métaux, présents en phase hétérogène, de la série du zinc, aluminium, titane, fer, chrome, cobalt et nickel.

8. Procédé selon la revendication 7, caractérisé par le fait que l'on utilise, par la scission thermique en phase liquide, un solvant dont le point d'ébullition est compris entre celui du 4,4'-diphénylméthane-diisocyanate et celui de l'alcool séparé.

9. Procédé selon la revendication 1 et 7, caractérisé par le fait que l'on utilise, comme catalyseur, de l'aluminium ou du zinc, qui est présent dans la phase hétérogène sous une forme à grande étendue superficielle.

10. Procédé selon la revendication 1 et 7, caractérisé par le fait que l'on scinde thermiquement en phase liquide le mélange de méthylène-bis-phényluréthanes et polyméthylène-polyphényluréthanes, on sépare par distillation une partie du diphénylméthane-diisocyanate obtenu, avec le solvant et on recycle avec celui-ci dans le réacteur de scission et l'on retire, comme fraction de dépôt, le mélange de la partie restante du diphénylméthanediisocyanate et du polyphényl-polyméthylène-polyisocyanate.